# EUROPEAN PATENT APPLICATION

(11) **EP 3 901 961 A1**
(43) Date of publication of application: **27.10.2021**
(21) Application number: 19934121.5
(22) Date of filing: 17.06.2019
(51) Int. Cl.: G16H 20/30, A61B 5/11, A61H 1/00

(54) **HARDWARE/SOFTWARE SYSTEM FOR THE REHABILITATION OF PATIENTS WITH COGNITIVE IMPAIRMENTS OF THE UPPER EXTREMITIES AFTER STROKE**

(71) Applicant: Limited Liability Company Sensomed, Moscow, 121205 (RU)
(72) Inventor: KAMOCKIJ, Andrej Sergeevich, Krasnoyarsk, 660021 (RU); FEDOROV, Aleksandr Vladimirovich, Krasnoyarsk, 660062 (RU)
(74) Representative: Berggren Oy
(86) International application number: PCT/RU2019/000431
(87) International publication number: WO 2020/256577

(57) **Abstract**

The invention relates to medicine. A hardware/software system for the rehabilitation of patients with cognitive impairments of the upper extremities after stroke comprises a virtual reality glove, a controller with a sensor, an encephalograph, and a computer with specialized cognitive games installed thereon. The glove is configured with built-in sensing elements that allow the movement of a patient's fingers and hand to be tracked in space. The controller is positioned on the shoulder joint in order to collect information about the movements of the shoulder joint during the execution of exercises in the games. The computer is configured to be capable of receiving information from the aforementioned devices; performing a computerized analysis of the information received regarding the patient's brain, arm muscle and fine motor skill function; determining the patient's fatigue level; automatically adjusting the exercises in the games and selecting the most effective exercises; and storing patient rehabilitation results in a database. The invention provides for more effective rehabilitation of patients with cognitive impairments of the upper extremities after stroke.

## Description

### FIELD OF THE INVENTION

This technical solution relates to a computer and medicine field, in particular, to the software and hardware system for rehabilitation of patients with upper limb post-stroke cognitive impairment.

### BACKGROUND

Every year 15 million of people in the world suffer a stroke, with about 5 million dead within several days and another 5 million become disabled. In 2010 r. about 33 million of people in the world lived with stroke sequelae. Cognitive impairment (CI) causes patient disability and social maladjustment and in some cases plays a larger part than movement disorders. Prevalence of post-stroke CI is high, on average, it occurs approximately in half of patients.

Post-stroke movement disorders occur in 80% of this case, they are one of the major causes of persistent disability and represent one of the global objectives of successive neurorehabilitation. Moreover, generally, upper limb functional recovery takes place at later date, often remaining the single cause of patient's disability.

Upper limb function rehabilitation is a labour-intensive process, quite often it takes months and years of focused efforts to recover arm global movements only, while hand movements, and, in particular, fine motor skills remain impossible, resulting in serious limitation of daily functioning. Ever-increasing amount of evidence-based data demonstrates that repeated, intensive training aimed at acquisition of skill improve upper limb recovery. However, sometimes, proceeding to skill acquisition training is impossible due to a barrier of insufficient arm movement level. Implementation of new automated and robotic devices, game strategies for arm recovery has opened new perspectives of arm paresis recovery due to using computer virtual strategies, activation of biofeedback, patient involvement.

It is common knowledge that human arm motor skills development is directly connected with development of interhemispheric specialty and interhemispheric interaction and, as a consequence, with development of speech, memory, attention and other cognitive functions. Analysis of the prior art demonstrates that individual elements of fine motor skills diagnostics are constituents of methods for assessment and control of human neuropsychological status peculiarities. Amount of information about arm fine motor skills obtained during such researches and defined by a specific task for implementation is, generally, limited.

The prior art solution discloses a cognitive impairment treatment in patients with cerebrovascular diseases (RU2268723C1, publ. 27.01.2006, Far Eastern National Medical University), this treatment comprises administration of microcirculatory and nootropic agents in the form of intravenous infusions within 10 days, and then tablet formulations of the same drugs are administered within 1 month and at the same time neuropsycological training is carried out which is aimed at improving social skills crucial for a patient, which are related to memorization of names, important dates, drugs and location of household items, with complication of 2 tasks as the patient makes progress; 30 minute training is carried out 3 times per week; course of treatment includes 12 trainings.

The known method of treatment supposes supervision and involvement of highly skilled professionals, and it cannot be carried out by a patient with no outside help, also it is used for patients with cerebrovascular pathology.

Besides, the prior art solution, RU2581707C1, publ. 20.04.2016, Viktor M. Shklovsky and colleagues., discloses a method of assessment of treatment and rehabilitation efficiency in patients with diminution of cerebral competence with brain lesions, including neuropsycological testing, which is carried out over time, before and after treatment and rehabilitation. Testing includes assessment of dominant verbal and nonverbal functions. Each test results are assessed on 1 to 10 scale, and they are used for determination of patient cerebral competence state dynamics during treatment and rehabilitation.

The disadvantages of this method are its labour intensity, necessity for large amount of stimulus material and special materials at hand for training, considerable time consumption, high requirements to qualification of neurologist or neuropsychologist. This method is not readily available for wide application since it requires involvement of highly specialized doctor - neuropsychologist, and it is essentially a diagnostic technique, but not a method for cognitive function correction.

Also, there is the prior art solution, which discloses a method for cognitive impairment correction at cerebrovascular pathology with the use of Schulte tables and noisy images (RU2506963C2, publ. 20.02.2014, State-Financed Educational Institution of the Higher Professional Education proff. V.F. Voino-Yasenetsky Krasnoyarsk State Medical University of Ministry of Health and Social Development of the Russian Federation), including providing a patient with an image of object on a "noisy picture" with a possibility of dosing the "noise" density, recognition of the given image by a patient, with image on a "noisy picture" displayed on a monitor, and at the same time a series of object images clearly depicted are additionally provided and on one of them there is an object from the "noisy picture", patient recognizes an object on the "noisy picture" by recognition and selection of the object from the provided variants, task is to be executed for set time, which maximum interval is defined by a doctor, and upon expiry of which the task is evaluated with demonstration of a score to a patient on the monitor, while the demonstrated score is directly proportional to the density of picture "noise", at which the patient gives a correct answer, and which is calculated by a formula, score demonstration is accompanied by visual and/or sound means, correction training is carried out within minimum 10 days, once per day, duration of one training is maximum 40 minutes.

The disadvantage of the known technical solution is unidirectionality of the invention for recovery of functions related to visual recognition and visual attention, lack of tasks training other higher cerebral functions, and also lack of tasks of diverse complexity, limited amount of stimulus material.

Besides, the prior art solutions of HandTutor, Meditouch (Israel) and Rapael smart glove, Neofect (Korea) are aimed at recovery of fine motor skills after neurological diseases at orthopedic diseases in postoperative period, at dystaxia.

However, the above solutions have limited functionality in terms of lack of possibility of movement pattern analysis, prediction of patient desired actions, it is not possible to collect analytics for all patients and store rehabilitation results in the analytics store database, it is not possible to involve patient's different parts of hand and wrist during rehabilitation.

The advantages of the claimed solution are as follows: when using the software and hardware system for rehabilitation of patients with upper limb post-stroke cognitive impairment the conditions for additional activation of motor cortex are created, game motivation is used, while the system is mobile, do not cause increase of hand spasticity, and it is capable of using by a patient with no outside help at home.

### SUMMARY OF THE INVENTION

The technical problem to be solved by the claimed technical solution is creation of the software and hardware system for rehabilitation of patients with upper limb post-stroke cognitive impairment, which is characterized in the independent claim. Additional variants of this invention implementation are presented in the subclaims.

The technical result is improving efficiency of rehabilitation of patients with upper limb post-stroke cognitive impairment.

The additional technical result achieved at implementation of the technical solution is reduction of rehabilitation time for patients with central nervous system damage and movement disorders, and reduction of doctor stressing.

Rehabilitation approach is based on brain plasticity and biological feedback. Brain recovery is closely connected with development of fine motor skills.

In the preferable embodiment the software and hardware system for rehabilitation of patients with upper limb post-stroke cognitive impairment is claimed, which comprises:
virtual reality glove (VR-glove) with integral sensing elements tracking movement of patient's fingers and hand in space;
controller with a sensor located on a shoulder joint to collect information about movements of the shoulder joint during exercising in task-specific games;
encephalograph for scanning patient's brain response during exercising in task-specific games by detection of electric pulses outgoing from its different areas;
computer with installed task-specific games which stimulate development of patient's arm motor functions, and the computer is configured to:
   - receive, from the above devices, information about movement of patient's fingers and arm, about brain activity during exercising in task-specific games;
   - perform deep computerized analysis of the obtained information about functioning of patient's brain, arm muscles and fine motor skills;
   - determine level of patient fatigability on the basis of the analyzed information, what game exercises improve patient rehabilitation and what exercises require correction;
   - correct automatically necessary game exercises and select the most effective exercises;
   - store patient rehabilitation results in the analytics store database (big data) after each gaming session.

In a particular embodiment VR-headset or glasses are additionally used.

In the other particular embodiment, selection of the most effective exercises, determination of fatigability level and assessment of the exercising result are carried out using artificial neural networks.

In the other particular embodiment, the software and hardware system additionally includes doctor and patient personal areas.

In the other particular embodiment, during cognitive games for rehabilitation a patient performs the following exercises:
a) finger flexion - extension;
b) spreading - approximation;
c) movements of individual fingers;
d) wrist rotation, hold.

In the other particular embodiment, encephalograph signal is prefiltered from extraneous signal and induced potentials.

### DESCRIPTION OF THE DRAWINGS

Implementation of the invention will be further described in accordance with the attached drawings, which are presented to clarify the invention chief matter and by no means limit the field of the invention. The following drawings are attached to the application:

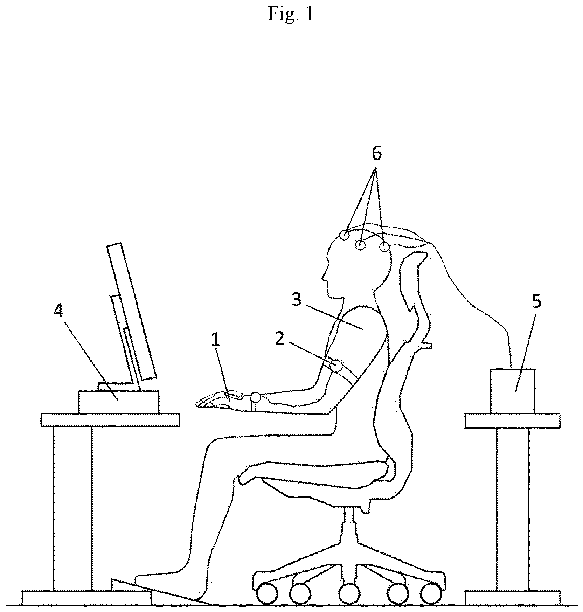

Fig. 1 illustrates a schematic view of rehabilitation process. Patient performs exercises on a computer using virtual reality glove and with connected encephalograph. Numbers in the view indicate the following:
   1. Virtual reality glove.
   2. VR-glove shoulder sensor.
   3. Patient.
   4. Computer with installed task-specific cognitive games.
   5. Encephalograph.
   6. Encephalograph electrodes connected to the patient.
Fig. 2 illustrates a schematic view of VR-glove components. Numbers in the view indicate the following:
   11. Glove (as material).
   12. Inertia sensors IMU comprising accelerometer, gyroscope and/or magnetometer.
   13. Vibrating motor transmitting sensation of vibration.
   14. System board.
   15. Battery.
   16. Connecting wires.
   17. Housing.
   18. Photodiod for integration with external optical tracking.

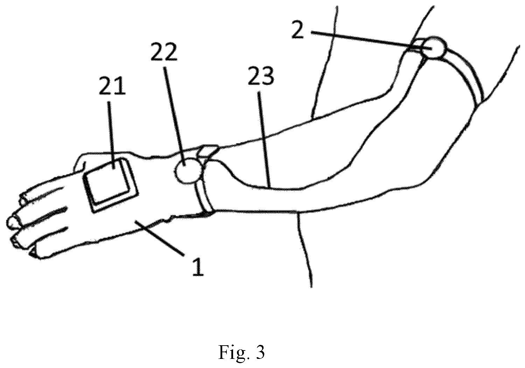

Fig. 3 illustrates VR-glove with a sensor on a shoulder joint. Numbers in the view indicate the following:
   1. Virtual reality glove.
   2. VR-glove shoulder sensor.
   21. VR-glove enclosure comprising system board and battery.
   22. VR-glove wrist sensor.
   23. Connecting wire.

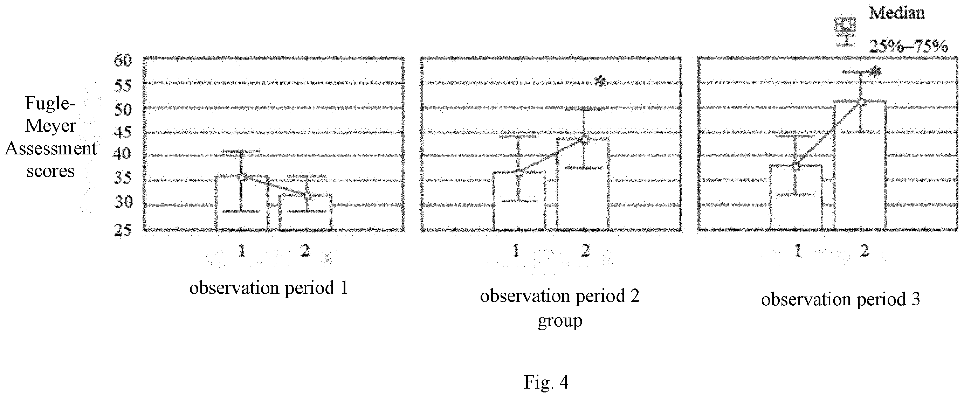

Fig. 4 illustrates upper limb motor function trends;

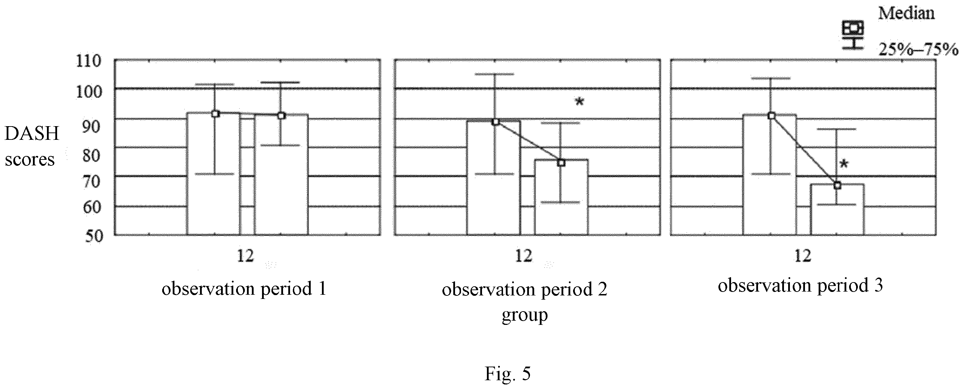

Fig. 5 illustrates improvement of daily activity scores.

### DETAILED DESCRIPTION OF THE INVENTION

Numerous implementation details intended to ensure clear understanding of this invention are listed in the detailed description of the invention implementation given next. However, it is obvious to a person skilled in the art how to use this invention as with the given implementation details as without them. In other cases, the well-known methods, procedures and components have not been described in details so as not to obscure the present invention.

Besides, it will be clear from the given explanation that the invention is not limited to the given implementation. Numerous possible modifications, changes, variations and replacements retaining the chief matter and form of this invention will be obvious to persons skilled in the art.

This invention is intended to create a software and hardware system for rehabilitation of patients with upper limb post-stroke cognitive impairment. This solution enables patients to perform exercises at home under doctor distant supervision. Since the claimed solutions additionally includes doctor and patient personal areas.

The claimed rehabilitation software and hardware system comprises such components as:
a) virtual reality glove (VR-glove) with integral sensing elements for finger and hand tracking;
c) encephalograph;
d) computer with installed task-specific cognitive games;
e) software reading and transmitting patient movements to the system;
f) a set of task-specific games for rehabilitation;
g) anonymized analytics store database (big data) for rehabilitation results suitable for analytics.

Patient rehabilitation in the claimed software and hardware system for patients with upper limb post-stroke cognitive impairment is performed by a set of task-specific cognitive games. Game control is based on certain gestures-exercises and fine motor skills.

Examples of exercises performed by a patient during games are given below:
1. finger flexion - extension;
2. spreading - approximation;
3. movements of individual fingers;
4. wrist rotation, hold.
5. wrist joint flexion, extension.

Types of muscles which functioning is subjected to in-depth examination are given below:
- Anterior arm muscles, superficial layer
   ∘ Round pronator muscle
   ∘ Radial flexor muscle of wrist
   ∘ Long palmar muscle
   ∘ Superficial flexor muscle of fingers
   ∘ Ulnar flexor muscle of wrist
   ∘ Brachioradial muscle
- Anterior arm muscles, deep layer
   ∘ Long flexor muscle of thumb
   ∘ Deep flexor muscle of fingers
   ∘ Quadratus pronator muscle
- Posterior arm muscles, superficial layer
   ∘ Long radial extensor of the wrist
   ∘ Short radial extensor of the wrist
   ∘ Extensor muscle of fingers
   ∘ Ulnar extensor muscle of wrist
   ∘ Extensor muscle of little finger
- Posterior arm muscles, deep layer
   ∘ Supinator muscle (forearm muscle)
   ∘ Long abductor muscle of thumb
   ∘ Short extensor muscle of thumb
   ∘ Long extensor muscle of thumb
   ∘ Extensor muscle of index finger
- Ball of thumb muscles
   ∘ Short abductor muscle of thumb
   ∘ Short flexor muscle of thumb
   ∘ Opposer muscle of thumb
   ∘ Adductor muscle of thumb
- Antithenar eminence muscles
   ∘ Abductor muscle of little finger
   ∘ Opposer muscle of little finger
   ∘ Long flexor muscle of little finger
   ∘ Short palmar muscle
- Middle hand muscles
   ∘ Lumbrical muscles
   ∘ Dorsal interosseous muscles of hand

Before to start game the patient places and fixes its hand in VR-glove with integral sensing elements for finger and hand tracking. Then he/she connects the glove to a computer, downloads and starts task-specific cognitive games aimed at development of motor functions. During rehabilitation the patient is to not just make movements but try to perform exercises correctly and on a regular basis.

Games, including virtual reality games, are developed with the use of Unitiy game engine. Unity —cross-platform environment for computer games development. Unity enables to create applications running with more than 20 different operating systems, including personal computers, game consoles, mobile devices, Internet applications, etc. The main advantages of Unity are availability of visual development environment, cross-platform support and modular component system. The capabilities are directed to creation of 3D games, including games for VR-glasses.

Provided that, the glove is used as a manipulator in such a way that the patient makes active movements of hand and fingers thereby enabling the set movements of the computer game object on the monitor and thereby causing actuation of sensing elements and their recording in the computer. The system provides for storing patient rehabilitation results in the analytics store database (big data) after each gaming session. Doctor has full access to data in the analytics store database, that afterwards enables to carry out objective tracking of the progress and compare it with other patients' collected data.

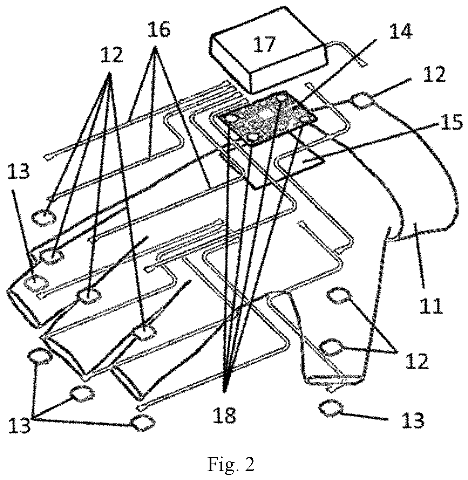

As detailed below in Fig. 2 the glove (11) is made of cloth inside which the following is placed:
(12) IMU sensors 8 pcs., each comprising accelerometer, gyroscope and/or magnetometer, 4 sensors are located on next to last phalanges of little finger, ring finger, long finger and index finger, 2 sensors are located on the first and second phalanges of thumb, 1 sensor is located on the system board, 1 sensor is located on wrist;
(13) vibrating motors, 5 pcs., one for each finger (on next to last phalanges of all fingers except for thumb and on the last phalanx of thumb);
(14) system board and computing module, Bluetooth radio module installed on the system board for communication with computer or other device via radio channel;
(15) battery;
(16) the required wiring for connecting sensors, vibrating motors, battery to the system board;
(17) system board and battery housing.

In contrast to analogues known in the prior art the VR-glove in the claimed solution can sense any movement of arm or finger in any direction, while the other products can track finger flexions only.

For neurorehabilitation tasks, where more precise monitoring of fine motor skills is required, the sensor system in the claimed solution is added by a controller with a sensor on a shoulder joint (2), and also by optical tracking.

As detailed below in Fig. 3, mutual arrangement of IMU sensors enables to resolve effectively the problem of "drift" of data on fingers and hand position in space and to track movements of a shoulder joint more effectively. In contrast to the systems with external tracking (based on photosensors or liquid emitting diodes and external cameras) this solution could be used without any external devices, that is convenient for working with mobile devices (smartphones).

Controller with sensor (2) could be also implemented in a wireless embodiment and be connected to computing module (21) via radio channel (Bluetooth orWiFi). In this case battery and radio module are located in the single module with controller (2).

The controller with a sensor is located on a shoulder joint to collect information about movements of the shoulder joint during exercising.

During gaming session, the patient could use VR-headset or glasses.

Arm muscular activity parameters measured during exercising are given below:
- Amplitude of muscular activity. Amplitude level indicates the level of neural signal.
- Rate of amplitude change. Rate of amplitude change provides us with detailed information about depth of cognitive bonds.
- Uncontrollable level of muscular activity. Measuring uncontrollable level of arm muscular activity enables to make a conclusion on time delay degree of corrective afferent signals. This parameter is also used to measure level of patient fatigability during exercising.
- Statistical analysis of dependent and independent span of variables and methods of descriptive statistics. Types of data distribution are evaluated by Shapiro-Wilk test.

Encephalograph is used for scanning patient's brain response during exercising in task-specific games by detection of electric pulses outgoing from its different areas. Encephalograph signal is prefiltered from extraneous signal and induced potentials. Moreover, using of encephalogram enables to conduct follow-up for such tasks as:
- automated selection of the most effective exercises;
- measurement of patient fatigability level.

Patient fatigability level plays an important role in effective rehabilitation of muscle-skeleton post-stroke cognitive impairment. Using of encephalograph together with artificial neural network enables to determine fatigability level to high precision. Moreover, the technique enables to associate brain activity with rehabilitation results and to automatize the procedure of selection of the most effective exercises for a patient.

During exercising to solve game tasks the data received from VR-glove, controller with sensor and encephalograph are transmitted in real time to the computer where in-depth computerized analysis of the obtained parameters of arm muscular activity, fine motor skills and brain activity is carried out. On the basis of the analyzed information, it is determined the level of patient fatigability, what game exercises improve patient rehabilitation and what exercises require correction. If necessary, the required game exercises are corrected and the most effective exercises are selected automatically. Selection of the most effective exercises, determination of fatigability level and assessment of the exercising result are carried out using artificial neural networks.

The principle of the technique is general training of new motor skills using virtual space. For example, the patient is suggested to control virtual arms or one arm by movements of legs and their fingers, by facial expression or shoulder girdle. Using of VR-glasses enables to create sufficient sense of reality for creation of new neural connections in brain motor part. In addition to training of general motor function, upon completion of exercise, the patient returns to reality and experience momentary stress of changing principles of own body control. It results in chaotic firing of motor neurons, among which there could be neurons controlling the damaged limb. Subsequently, it serves as a primary basis for creation of new biological neural connections controlling the muscle-skeleton part of concern bypassing the brain damaged part.

The technique used in the claimed neurorehabilitation solution enables to obtain data on exact position and movements of fingers, wrists and forearms in space and to transmit these data in real time for using in computer.

On a computing device, for example, computer there is installed a software-driver which uses the sensors data (angular velocities and acceleration vectors) and converts them into space turn quaternions for the following joints: turn (flexion) of finger last phalanx relative to palm for index, long, ring fingers and little finger in vertical plane relative to palm; turn of index, long, ring fingers and little finger in horizontal plane (palm plane); turn/flexion of thumb relative to palm in space; turn/flexion of hand (palm) relative to elbow joint; turn of elbow joint relative to shoulder joint.

Then, based on the obtained angles, turns of all other joints and relative position of all hand joints are computed using inverse kinematics algorithms (data on joints connections and their linear dimensions).

Efficiency of the claimed solution is supported by studies carried out involving project team members. 43 patients admitted to neurorehabilitation course in Siberian Clinical Center of Federal Medical-Biological Agency of Russia, Krasnoyarsk, took part in the study. The patients were randomized into two groups.

Main group - 20 people, (10 men, 10 women), aged 44 to 74 (median age - 61).

Control group - 23 people, (17 men, 6 women), aged 46 to 76 (median age - 59).

All patients suffered hemispherical ischemic stroke 6 to 24 months ago, the diagnosis was confirmed by the results of brain MRT.

For the purpose of statistic processing of neurological examination results the following scales were filled in: NIHSS - neurological rating, modified Ashworth scale for arm spasticity assessment, set of scales for assessment of upper limb functional capabilities: Motor Assessment Scale, subsections F - upper limb global motor skills, G - hand movements, H - hand fine motor skills. Fugl-Meyer Assessment scale, 9 Hole peg test were used for assessment of upper limb movement disorders; DASH scale was used for assessment of daily functioning disorders caused by upper limb paresis. Hospital Anxiety and Depression Scale was used for objectivization of emotional-volitional disorder level.

In addition to extensive neurological examination all patients were subjected to paraclinic follow-up examination for defining background disease more exactly, clinical and biochemical blood and urine analyses, ultrasonography of brachiocephalic arteries, electrocardiographic examination, therapeutist examination.

At study entry the groups did not differ in sex, age, stroke severity, hand spasticity intensity, level of hand fine motor skills, level of emotional-volitional disorders, severity of brachiocephalic arteries stenosis, severity of comorbidity.

In the main group in addition to standard hospital treatment the patients were given training of hand fine motor skills using the claimed system once per day for 40-60 minutes, 5 days per week, within two weeks.

In the control group the patients were given only standard treatment which included: drug therapy, instructor session for passive, active training of upper limb movements, massage of upper limb muscles, exercises for recovery of hand fine motor skills in the form of patient self-training after instructions given by rehabilitation physician/instructor, within two weeks for 40-60 minutes per day, 5 days per week.

After the treatment course hand movement disorders and daily functioning level were reassessed. Besides, the scores of NIHSS, Ashwort and HADS scales were follow-up assessed.

Statistic processing of study data was carried out using application software package Statistics, 7 (Statsoft, USA). Normality assessment was carried out using Kolmogorov-Smirnov test. The results of comparison in independent groups were assessed using Mann-Whitney nonparametric statistics, differences at p <0.05 were considered statistically significant. Analysis in dependent groups was carried out using Wilcoxon nonparametric statistics. Attribute comparisons were carried out according by χ² criterion. Correlations were assessed using Spearman's nonparametric test.

After treatment course in both groups there were recorded statistically significant improvements in scores of neurological status, emotional-volitional disorder level, spasticity intensity, level of upper limb movement disorders by Motor Assessment Scale (Table 1, 2).

Besides, in the main group there were recorded additional changes by 9 Hole peg test for paralyzed arm, and also by DASH test subscales (Table 2), that is illustrative of achieved higher level of daily functioning after additional use of the claimed system as a rehabilitation technique.

**Table 1**

| Scores of upper limb motor activity, psychometric scales and degree of daily functioning limitation before and after treatment course (14th day of therapy) in the control group. | | | | |
|---|---|---|---|---|
| Assessed parameters | Number of follow-ups | Before treatment Me (Q₂₅; Q₇₅) | After treatment Me (Q₂₅; Q₇₅) | P |
| NIHSS scale | 28 | 5 (4; 6) | 4 (3; 5) | 0.005* |
| Ashworth scale | 28 | 2 (1; 2) | 2 (1; 2) | 0.108 |
| MMSE scale | 29 | 27 (25; 28) | 28 (26; 29) | 0.001* |
| HADS scale (depression subscale) | 29 | 4 (3; 5) | 4 (4; 5) | 0.044* |
| HADS scale (anxiety subscale) | 29 | 4 (3; 6) | 4 (3; 5) | 0.059 |
| FMA scale | 29 | 36 (29; 41) | 37 (31; 44) | <0.001* |
| 9 Hole peg test | 28 | 128 (102; 224) | 131 (102; 212) | 0.135 |
| MAS scale (shoulder section) | 29 | 4 (3; 6) | 6 (4; 6) | 0.005* |
| MAS scale (forearm section) | 29 | 4 (3; 4) | 4 (3; 4) | 0.067 |
| MAS scale (hand section) | 29 | 2 (1; 3) | 2 (1; 3) | 0.001* |
| MAS scale (general) | 29 | 10 (7; 13) | 11 (9; 13) | <0.001* |
| DASH scale | 29 | 92 (71; 102) | 89 (71, 105) | 0.885 |

| | | | | |
|---|---|---|---|---|
| Note: ^{∗} - changes were considered statistically significant at p<0.05. | | | | |

**Table 2**

| Scores of upper limb motor activity, psychometric scales and degree of daily functioning limitation before and after treatment course (14th day of therapy) in the main group. | | | | |
|---|---|---|---|---|
| Assessed parameters | Number of follow-ups | Before treatment Me (Q₂₅; Q₇₅) | After treatment Me (Q₂₅; Q₇₅) | P |
| NIHSS scale | 20 | 6 (4.5; 7) | 4 (3; 4) | <0.001* |
| Ashworth scale | 20 | 2 (1; 2) | 1 (1; 2) | 0.041* |
| MMSE scale | 20 | 28 (27; 29) | 2* (27; 29) | 0.182 |
| HADS scale (depression subscale) | 20 | 3 (2; 5.5) | 3 (2; 3.5) | 0.342 |
| HADS scale (anxiety subscale) | 20 | 4 (3.5; 6.5) | 4 (2.5; 5) | 0.789 |
| FMA scale | 20 | 32 (29; 36) | 43.5 (37.5; 49.5) | <0.001* |
| 9 Hole peg test | 20 | 102 (102; 181.5) | 102 (67.5; 159.5) | 0.026* |
| MAS scale (shoulder section) | 20 | 4 (2.5; 6) | 5.5 (5; 6) | 0.005* |
| MAS scale (forearm section) | 20 | 3 (2; 4) | 5 (4; 6) | <0.001* |
| MAS scale (hand section) | 20 | 2.5 (1; 4) | 4 (3.5; 5.5) | <0.001* |
| MAS scale (general) | 20 | 9.5 (8; 13) | 14.5 (12; 17) | <0.001 |
| DASH scale | 20 | 91.5 (80.5; 102.5) | 75.5 (61.5; 88.5) | <0.001* |

| | | | | |
|---|---|---|---|---|
| Note: ^{∗} - changes were considered statistically significant at p<0.05. | | | | |

When comparing treatment groups after therapy course there were recorded statistically significant differences in general level of the Motor Assessment Scale, upper limb subsection, and, in particular, hand movement and fine motor skills subscales, and also statistically significant improvement of daily functioning scores (p <0.05).

Training with the use of the claimed system considerably increased patient motivation and attitude for recovery. As a consequence - there were recorded differences in groups by HADS depression subscale level (Table 3).

**Table 3**

| ***Scores of upper limb motor activity, psychometric scales and degree of daily functioning limitation in the main* and *control groups after treatment (Mann-Whitney test)*** | | | | | | |
|---|---|---|---|---|---|---|
| Assessed parameters | Before treatment | | | After treatment | | |
| | Control group | Main group | p-level | Control group | Main group | p-level |
| NIHSS scale | 5 (4; 6) | 6 (4.5; 7) | 0.122 | 4 (3; 5) | 4 (3; 4) | 0.366 |
| Ashworth scale | 2 (1; 2) | 2 (1; 2) | 0.726 | 2 (1; 2) | 1 (1; 2) | 0.304 |
| MMSE scale | 27 (25; 28) | 2* (27, 29) | 0.194 | 28 (26; 29) | 28 (27; 29) | 0.604 |
| HADS scale (depression subscale) | 4 (3; 5) | 3 (2; 5.5) | 0.073 | 4 (4; 5) | 3 (2; 3.5) | <0.001** |
| HADS scale (anxiety subscale) | 4 (3; 6) | 4 (3.5; 6.5) | 0.932 | 4 (3; 5) | 4 (2.5; 5) | 0.951 |
| FMA scale | 36 (29; 41) | 32 (29; 36) | 0.353 | 37 (31; 44) | 43.5 (37.5; 49.5) | 0.105 |
| 9 Hole peg test | 128 (102; 224) | 102 (102; 181.5) | 0.315 | 131 (102; 212) | 102 (67.5; 159.5) | 0.117 |
| MAS scale (shoulder section) | 4 (3; 6) | 4 (2.5; 6) | 0.377 | 6 (4; 6) | 5.5 (5; 6) | 0.951 |
| MAS scale (forearm section) | 4 (3; 4) | 3 (2; 4) | 0.260 | 4 (3; 4) | 5 (4; 6) | <0.001* |
| MAS scale (hand section) | 2 (1; 3) | 2.5 (1; 4) | 0.111 | 2 (1; 3) | 4 (3.5; 5.5) | <0.001* |
| MAS scale (general) | 10 (7; 13) | 9.5 (8; 13) | 0.875 | 11 (9; 13) | 14.5 (12; 17) | <0.001* |
| DASH scale | 92 (71; 102) | 91.5 (80.5; 102.5) | 0.281 | 89 (71, 105) | 75.5 (61.5; 88.5) | 0.024* |

| | | | | | | |
|---|---|---|---|---|---|---|
| *Note:* ^{∗} - *changes were considered statistically significant at p <0.05.* | | | | | | |

When assessing results of the follow-up observation, the disorder severity and functioning level were statistically significantly better in the group rehabilitated with the use if the claimed system (Table 4).

**Table 4**

| ***Scores of upper limb motor activity, psychometric scales and degree of daily functioning limitation in the main and control groups in catamnesis (Mann-Whitney test)*** | | | |
|---|---|---|---|
| Assessed parameters | Main group Me (Q25; Q75) | Control group Me (Q25; Q75) | P |
| NIHSS scale | 4 (3; 4) | 4 (3; 5) | 0.437 |
| Ashworth scale | 1 (1; 2) | 1 (1; 2) | 0.818 |
| MMSE scale | 28.5 (28, 29.5) | 28 (26; 29) | 0.141 |
| HADS scale (depression subscale) | 4 (2; 5.5) | 5 (3; 7) | 0.230 |
| HADS scale (anxiety subscale) | 4.5 (3; 6) | 4 (2; 7) | 0.604 |
| FMA scale | 51 (45; 57) | 38 (32; 44) | <0.001* |
| MAS scale (shoulder section) | 6 (5; 6) | 6 (5; 6) | 0.377 |
| MAS scale (forearm section) | 6 (5; 6) | 4 (3; 5) | <0.001* |
| MAS scale (hand section) | 4 (4; 5.5) | 2 (1; 3) | <0.001* |
| MAS scale (general) | 16 (14.5; 17) | 11 (10; 14) | <0.001* |
| DASH scale | 67.5 (60.5; 86) | 91 (71; 104) | <0.001* |

| | | | |
|---|---|---|---|
| *Note:* ^{∗} - *changes were considered statistically significant at p <0.05.* | | | |

As Table 4 shows, the main group patients demonstrated additional improvement by upper limb functioning scales (Fugle-Mayer Scale, Motor Assessment Scale), besides, there was statistically significant improvement of daily activity associated with upper limb, and emotional background was also improved by HADS depression subscale.

Upper limb motor function trends are shown in Fig. 4. Upper limb motor function trends by Fugle-Meyer Assessment scale in patients of the main (2) and control (1) group at study entry, at the end of two-week therapy course and in catamnesis. X-direction - study groups, Y-direction ― Fugle-Meyer Assessment scale scores.

Note: - changes were considered statistically significant at p <0.05.

Analysis of daily functioning trends in relation to upper limb function disorder has led us to a conclusion about significant effect of the used system on improving daily activity scores (Fig. 5). Upper limb disability score trends in patients of the main (2) and control group at study entry, at the end of two-week therapy course and after 6 months of follow-up observation. X-direction - study groups, Y-direction ― DASH scale scores.

Note: ^{∗} ― changes were considered statistically significant at p <0.05.

As Fig. 5 shows, decrease of upper limb disability scores is observed in catamnesis, which indicates high functional recovery of upper limb function, patient reintegration to daily living activity.

Results of the study data correlation analysis by Spearman's rank method ascertain the existence of certain interrelations. Moderate association is defined between the attributes:
- sex and USDG data: men more often suffered brachiocephalic arterial occlusive disease (direct, moderate, r = 0.46, p <0.05);
- vascular pool and spasticity scores (at left vascular pool disease the Ashworth scale score was higher, interrelation - moderate, (r = 0.33, p <0.05), hand function level - lower - reverse, moderate (r = -0.34, p <0.05) and follow-up level of diffidence caused by hand disorders - higher (direct, moderate, r = 0.36, p <0.05);
- ultrasonography of cervical vessels and upper limb disability indices (direct, moderate, r = 0.46, p <0.05)
- scores of neurological status disorder severity by NIHSS scale and degree of upper limb functional capabilities by FMA scale, (r = -0.38, p <0.05, reverse, moderate),
- MMSE scores and level of upper limb functional capabilities by FMA scale (r = 0.46, p <0.05, direct, moderate)

The following scores have been correlated to high follow-up scores of upper limb persisting disability (DASH):
- severity of brachiocephalic arteries stenosis (r = -0.32, p <0.05, reverse causality, moderate),
- level of hand fine motor skills at study entry by MAS scale (r = -0.34, p <0.05, reverse causality, moderate),
- severity of neurological defect at study entry (r = 0.32, p <0.05, direct causality, moderate).

The study results make it possible to state that using the software and hardware system for rehabilitation of patients with upper limb post-stroke cognitive impairment is a highly efficient technique as compared to standard post-stroke therapy, which has an effect not only on disorder level, but also on enhancement of daily use of paralyzed hand. Besides, this technique is interested for a patient, it improves motivation for training through patient involvement into training process.

The follow-up observation data make it possible to consider that recovery of hand fine motor skills sharply increases level of using paretic limb, that, in turn improves functional recovery and daily activity.

In case of evident hand paresis, it is useful to involve unaffected limb by training with use of two gloves - on unaffected and paralyzed limb.

The advantage of the claimed system is speeding up of patient rehabilitation.

The following advantages are additionally achieved at implementation of the claimed software and hardware system for patient rehabilitation:
- analysis of patient movement patterns, prediction of patient desired actions;
- availability of biofeedback for different body segments: forearm, shoulder, paretic leg, etc. with combining them into a single system;
- measuring of finger swing in any direction by any quantity;
- measuring of palm turn and swing in any direction;
- measuring of hand, wrist movement and turn in any direction by any quantity;
- analysis of fine motor skills;
- smooth (indiscrete) measuring of movement parameters;
- measuring of passive movement range;
- measuring of active movement range;
- measuring of finger movement speed;
- movement quality characteristic such as trembling or fluidity;
- monitoring of patient rehabilitation progress;
- collecting of analytics for all patients and storing of rehabilitation results in the analytics store database.

The application materials have represented the preferred embodiment of the claimed technical solution, which shall not be used as limiting the other particular embodiments, which are not beyond the claimed scope of protection and are obvious to persons skilled in the art.

## Claims

1. Software and hardware system for rehabilitation of patients with upper limb post-stroke cognitive Impairment comprising:
virtual reality glove (VR-glove) with integral sensing elements tracking movement of patient's fingers and hand in space;
controller with a sensor located on a shoulder joint to collect information about movements of the shoulder joint during exercising in task-specific games;
encephalograph for scanning patient's brain response during exercising in task-specific games by detection of electric pulses outgoing from its different areas;
computer with installed task-specific games which stimulate development of patient's arm motor functions, and the computer is configured to:
• receive, from the above devices, information about movement of patient's fingers and arm, about brain activity during exercising in task-specific games;
• perform deep computerized analysis of the obtained information about functioning of patient's brain, arm muscles and fine motor skills;
• determine level of patient fatigability on the basis of the analyzed information, what game exercises improve patient rehabilitation and what exercises require correction;
• correct automatically necessary game exercises and select the most effective exercises;
• store patient rehabilitation results in the analytics store database (big data) after each gaming session.

2. System of claim 1 **characterized in that** VR-headset or glasses are additionally used.

3. System of claim 1 **characterized in that** selection of the most effective exercises, determination of fatigability level and assessment of the exercising result are carried out using artificial neural networks.

4. System of claim 1 **characterized in that** it additionally includes doctor and patient personal areas.

5. System of claim 1 **characterized in that** during cognitive games for rehabilitation a patient performs the following exercises:
a) finger flexion - extension;
b) spreading - approximation;
c) movements of individual fingers;
d) wrist rotation, hold.

6. System of claim 1 **characterized in that** encephalograph signal is prefiltered from extraneous signal and induced potentials.
